# FASCICULE DE BREVET EUROPEEN

(11) **EP 1 608 381 B1**
(45) Date de publication et mention de la délivrance du brevet: **21.11.2007**
(21) Numéro de dépôt: 03717400.0
(22) Date de dépôt: 13.02.2003
(51) Int. Cl.: A61K 33/30, A61K 31/05, A61K 31/01, A61K 31/235, A61K 36/81, A61P 17/02, A61P 35/00

(54) **COMPOSITIONS COSMETIQUES ET PHARMACEUTIQUES CONTENANT DU LYCOPENE**
KOSMETISCHE UND PHARMAZEUTISCHE ZUSAMMENSETZUNGEN MIT LYCOPIN
THERAPEUTIC AND CONSMETIC APPLICATION FOR A COMPOSITION CONTAINING LYCOPENE

(30) Priorité: 15.02.2002 FR 0201956; 20.12.2002 FR 0216425
(43) Date de publication de la demande: 28.12.2005
(73) Titulaire: Touche, Marie-José, 75016 Paris (FR)
(72) Inventeur: Touche, Marie-José, 75016 Paris (FR)
(86) Numéro de dépôt international: PCT/FR2003/000471
(87) Numéro de publication internationale: WO 2003/068200

(56) Documents cités:
- EP-A- 0 986 963
- WO-A-01/85182
- WO-A-01/89542
- WO-A-02/05827
- WO-A-92/00085
- WO-A-94/14412
- FR-A- 2 770 974
- FR-A- 2 790 389
- FR-A- 2 806 262
- US-B1- 6 258 855

## Description

### A:Le Butoforme ou Scuroforme, associé au produit B:L'eugénol et au produit C:L'oxyde de zinc, additionné de Lycopène

Le mélange des 2 produits **B** et **C** représentant **l'eugénate**

Le mélange des 3 produits **A,B,C,** possède une action renforcatrice et potentialisatrice des actions déjà mutuellement exaltées par les composants de **A+B+C,** auquel la demanderesse apporte l'adjonction d'un produit **D : Un colorant alimentaire rouge : le Lycopène** contenu dans l'oléorésine de tomate produit naturel à 100% de tomate.

Il s'agit d'une synergie de potentialisation, entre l'association d'un anesthésique local : le butoforme-scuroforme sous forme de base libre ou d'un sel pharmaceutiquement acceptable,en mélange avec un eugénate composé d'un produit A : **l'eugénol**, malaxés avec l'oxyde de zinc pour obtenir une rémission immédiate de la douleur aigue ou chronique, qui résulte d'une stimulation forte des récepteurs de la douleur sans lésions, ni dysfonctionnement du système nerveux périphérique et central.

Le mélange obtenu **A,B,C,D**, antalgique, supprime l'inflammation et l'infection, tout en favorisant l'hémostase et la cicatrisation tissulaire et osseuse.

### Cette application thérapeutique concerne l'homme et l'animal

### Le produit A :

Il s'agit d'un anesthésique local : appelé encore Planoform, Butoform (D.C.F) Butylbeloform **ou Scuroforme ou BUTAMBEN (D.C.L) ou p-aminobenzoate de n- butyle ( D.S), selon la pharmacopée francaise.ou le scuroforme.**

Cet anesthésique local a été sélectionné à partir d'un groupe d'anesthésiques locaux :la procaine, la lidocaine, la prilocaine, la mupivicaine, la dycloine, la dibucaine, la benzoine, la chloroprocaine, la tetracaine, la bupivacaine, l'etidocaine.

Il représente le principe actif, sous forme de base libre ou d'un sel pharmaceutiquement acceptable. Les anesthésiques locaux sont des sels organiques fortement ionisés, leur pénétration est plus facile si la base est dissoute dans un milieu gras.

### Propriétés-Indications :

Anesthésique local utilisé sur la peau, les muqueuses et la cornèe.
en rhinolaryngologie, en gastro-entérologie,et en dermatologie.

### Les caracteristiques du Butoforme sont :

Caractères organoleptiques : poudre cristalline, blanche, inodore, insipide, fusible à 57-58°. Le point de fusion du picrate est compris entre 109°-110°; pratiquement insoluble dans l'eau froide, mais facilement soluble dans le chlorure de méthylène,soluble dans 2p.d'alcool à 95°, 10 p.d'alcool à 50°, 12,5 p.5 d'huile d'olive, 200p de vaseline.

**Formule chimique du Butyl-4-aiminobenzoate,** qui est un ester butylique de l'acide para-aminobenzoique
=benzoic acid, 4-amino-, butyl ester ;(2)Butyl p. amino benzoate
de formule (C11H15NO2)2C6H3N3O7.615,60
composé de deux molécules A et une molécule de B.
**A** représente le benzoic acid ,- amino-, butylester, et **B** composé de 2, 4, 6, trinitrophénol (2 : 1)

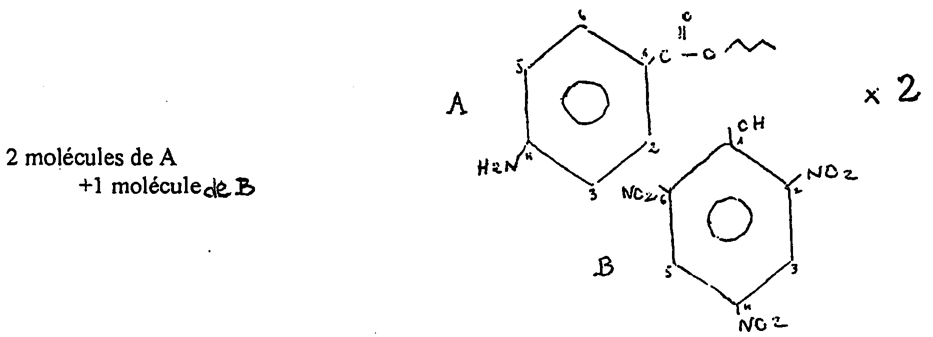

| | **NORMES** | **RESULTATS** |
|---|---|---|
| **Identité du point de fusion** | 57°C a 58°C | 58°C |
| **Coloration** | Blanche à infime | CONFORME |
| **Odeur** | Nulle à infime | CONFORME |
| **Spectre infrarouge** | Identique au spectre de référence | CONFORME |
| **Spectre ultraviolet** | Maximum 292 à 294 nm | Max=293nm |
| | Minimum 239 à 243 nm | Min= 241nm |
| **Examen CCM** | 0 | 0 |
| **-Impureté >1%** | | |
| **Solution éthanolique à 10%** | Limpide | Limpide |
| **Coloration de cette solution** | DO(450 nm) : 0.030 | DO 450nm=0.007 |
| **Cendres sulfuriques** | <ou= 0.1% | CONFORME |
| **Metaux lourds** | 0.0010% Max | CONFORME |
| **Titre % sur sec** | 99 à 101% | 100.86% |
| **(diazoation)** | | |
| **Solution dans l'ether** | Limpide | Limpide |
| **1g/30ml** | | |

les dérivés du butoforme : le seul sel du BUTOFORM est le Butamben Picrate (C11H15NO2)2 C6H3N3O7.615.60 Benzoic acid, 4-amino-,butyl ester, composé de 2,4,6 trinitrophenol (2 : 1)

Anesthésique local topique pour la peau et les muqueuses, le lactose-butoforme est un produit totalement résorbable, mais si utilisation 10%, le produit devient semi-résorbable.

### LE PRODUIT B : L' EUGENOL :

### -Nature chimique du produit :

### Composition qualitative et quantitative :

Principe actif de l'essence de girofle qui en contient 96% (titrage 96 sur 100) n'est pas un produit de synthèse.
incolore à jaune pâle, insoluble dans l'eau, soluble dans l'éthanol acétate d'eugényle et B-caryophyllène (environ 10%) en petites quantités.

Composé aromatique dérivé du phényl propane issu d'une des trois classes de principe actif des huiles essentielles en aromathérapie. L'eugénol posséderait des propriétés anticarcinogènes, grâce aux sesquiterpènes en C15 des clous de girofle.

### Autres constituants :

Flavonoides (dérivés du quercétol et du kaempférol) ; tanins ; acides carboxyphénoliques (acides gallique,protocatéchique); traces de stérols et d'hétérosides :10% d'huile grasse.

Forme pharmaceutique :
liquide .....
Classe pharmaco-thérapeutique cariophyllène eugénol :R=OH
acétate d'eugényle R=COCH:3:
   de formule 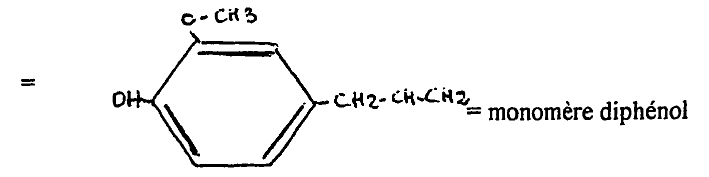
caractérisée par un noyau benzénique portant en ortho une fonction phénol OH libre (potentiel acide tres faible), et une autre fonction méthoxyle OCH3 en para, saturée par radical CH3. L'existence et la disposition de ces 2 fonctions indispensables au phénomène de prise.

La différence entre l'eugénol et l'essence pure est la rapidité de durcissement avec l'eugénol pur.

### 2°Indications thérapeutiques :

- L'eugénol est un antiseptique et antibactérien, un antifungique, un antiviral, un spasmolytique, un topique dentaire (huile essentielle pure)et un léger anesthésique local, pour l'inflammation et les lésions de la muqueuse buccale (solution de 1 à 5 % d'huile essentielle) inflammation et lésions de la muqueuse buccale (solution de 1 a 5 % d'huile-essentielle).
- L'eugénol est utilisé dans le domaine de l'art dentaire et au niveau du pharynx.

### 3°Interactions médicamenteuses :

aucune, selon la commission E. (1985)voir dictionnaire Uzan

### -contre-indications :

pas de contre indication a ce jour.

### -Effets secondaires :

Irritation des tissus, sous forme concentrée. L'huile peut être diluée dans une huile de base, telle que l'huile d'olive, de germe de blé, d'amande douce, de tournesol, de pépins de raisin, d'huile de bourrache ou d'onagre ou de soja : 5 à 10 gouttes d'huile essentielle pour 20 ml d'huile de base de culture biologique. Il se produit ,dans ce cas, une influence sur la dureté du produit final, qui reste malaxable pendant plusieurs jours.

### LE PRODUIT C :

### L'OXYDE DE ZINC =Z N O basique

Autre dénomination : flores de zinc, zinc oxyde, blanc de zinc, sel ou dérivé = zinc peroxyde.
se présente sous forme de poudre blanchâtre, sans odeur, point d'intervalle de fusion : 1970°C de densité relative de 5,6 insoluble dans l'eau , soluble dans les acides et dans les bases.
- L'oxyde de zinc est : un astringent, un antiseptique externe, un antiseptique. Il est inhibiteur de l'activateur du plasminogène. Le plasminogène est un ferment du plasma, transformé, pouvant activer la prothrombine en l'absence de calcium sous l'action d'une kinase d'origine tissulaire ou bactérienne :
   L'eugénate et son acétate sont inhibiteurs de l'agrégation thrombocytaire.

Le Zinc possède deux électrons sur sa couche périphérique :
Rôle avec le magnésium.

Le Zinc est essentiel pour la synthèse de l'ADN, des protéines, de l'insuline et du métabolisme des acides gras polyunsaturés, il est nécessaire pour la reproduction, la maturation sexuelle, la croissance, la cicatrisation et protège contre les radicaux libres.

Au contact de l'air atmosphérique, l'oxyde de zinc rencontre deux possibilités d'altération invisibles macroscopiquement.
- Avec l'humidité, ZNO s'hydrolyse et donne l'hydroxyde de zinc qui conserve sa fonction basique.
   ZNO+H2O |donne ZN(OH2) donc ZNO peut être ZNO, ou ZN(OH)2
- Le mélange de l'eugénol et de l'oxyde de zinc forme **l'EUGENATE,** qui est une composition sous forme de pâte, composée de 70 à 80% d'=oxyde de zinc pulvérulent et de quelques gouttes d'eugénol, comprenant une concentration de 2 D C d'oxyde de zinc +3 gouttes d'eugénol, jusqu'à obtenir une pâte homogène lisse et rigide, compatible avec une certaine élasticité, permettant de réaliser des bords du pansement non coupants, donc non irritants, d'aspect lisse, pour ne pas blesser les tissus mous, avec néanmoins une résistance du pansement à la traction et au cisaillement.

Ces qualités confèrent au patient un confort les jours suivants l'intervention et une protection de la plaie vis à vis du milieu extérieur.

La réaction de prise de l'eugénate répond à la neutralisation de la fonction phénol (acide tres faible )de l'eugénol, par la base ZNO.

L'eau H2O sert de catalyseur au ciment Eugénol + ZNO.

La fonction phénol ( acide très faible) est incapable de déplacer CO2 dans le carbonate de zinc (formé au contact du CO2 atmosphérique) : si l'oxyde de zinc est en partie carbonaté, le carbonate immobilise une partie de l'eugénol (ralentissement de la prise de 48 heures).

Si on chauffe ZNO, on le retrouve pur avec les mêmes affinités et une plus grande rapidité de prise : CO3ZN et ZN(OH) 2.

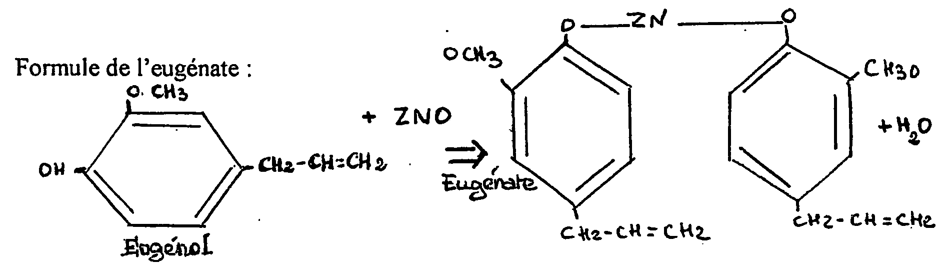

L'Eugénol est un aldéhyde ayant une fonction phénol libre et une autre saturée par radical CH3

L'eau sert de catalyseur à l'eugénate qui durcit de la surface en profondeur.

La fonction phénol ne peut pas déplacer le CO2 du CO3ZN.

Si ZNO est chauffé (600 degrés), on retrouve ZNO pur.

Depuis la demanderesse a trouvé que l'on pouvait ajouter du lycopène pour améliorer l'esthétique tissulaire, et la cicatrisation des tissus mous et osseux, permettant d'obtenir un comblement, lors des pertes tissulaires pathologiques ou chirurgicales.

Le mélange des produits **A+B+C** représente une composition antalgique et analgésique, capable de supprimer l'inflammation et l'infection et de favoriser l'hémostase.

### LE PRODUIT D : LE LYCOPENE.

Le lycopène, appelé encore All-trans-lycopène, cis-lycopène, psi-psi-carotène et est un pigment rouge présent dans certains végétaux et algues est issu de la peau des tomates fait partie des caroténoides qui sont des composés phénoliques et plus précisément des terpènes : pigments jaunes orangés présents dans les fruits et légumes. Le lycopène est l'un des six prédominants caroténoides apparaîssant dans le plasma humain. Le lycopène est le plus puissant antioxydant des 600 caroténoides de la nature. Le lycopène procure une stratégie efficace pour prévenir les dommages oxydatifs et peut contribuer à réduire la perte osseuse. Il limite le vieillissement prématuré, diminue le risque de dégénérescence de la macula, réduit la formation de la plaque athéromateuse dans les vaisseaux sanguins, diminuant ainsi le risque des troubles cardiaques. Il inhibe la croissance des cellules cancéreuses et réduit l'oxydation du mauvais cholestérol. Le lycopène est efficace contre l'oxygène singulet, radical libre connu pour son action sur la destruction cellulaire. Le lycopène a 2 fois plus d'effet sur les radicaux libres que le Bétacarotène (précurseur de la vitamine A). Il a un effet protecteur sur plusieurs types de cancers. Il prévient la formation des tumeurs et diminue la progression des tumeurs existante.

Sa biodisponibilité est fonction de nombreux facteurs, tels que le broyage et la cuisson.

Le lycopène est peu sensible à la chaleur, ne présente pas de contre indication ou d'effet secondaire.

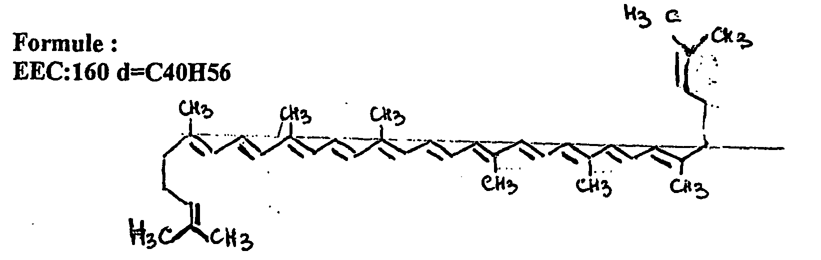

Le lycopène est un caroténoide acyclique avec 11 doubles liaisons en configuration trans, mais des isomères cis existent dans les produits transformés issus de tomates et qui ont une meilleure biodisponibilité, car ils sont plus solubles dans les sels biliaires et mieux incorporés dans les chylomicrons.

Utilisation : sous forme d'oléorésine : liquide visqueux colorant.

Le lycopène est ajouté pour son rôle d'antioxydant et pour améliorer l'esthétique tissulaire.

### PRODUIT FINAL :

**3.METHODE de préparation du produit final A, B, C , D,** pour préparer un médicament destiné à obtenir une rémission de la douleur aigue ou chronique et une réparation tissulaire et osseuse. La préparation est fabriquée à base de cristaux de butoforme dissous dans l'eugénol, auxquels on ajoute l'oxyde de Zinc, par malaxage jusqu'à obtenir une consistance mastic. On additionne 0,05 % d'oléorésine de tomate contenant du lycopène, ou un dérivé pharmaceutiquement acceptable, en tant que colorant et anti-oxydant afin d'obtenir la coloration tissulaire souhaitée, pour améliorer l'esthétique tissulaire. Une protection à base de corps gras de type vaseline est souhaitable autour du produit qui peut présenter un léger picotement de surface.

Le pansement sera renouvelé de préférence tous les 4 jours afin d'obtenir le maximum d'efficacité.

La composition A+B+C+D, formée d'Oxyde de zinc, de Scuroforme, d'Eugénol et de Lycopène est mélangée dans les proportions idéales suivantes :
- 1/16 CG de cristaux de Butoforme-Scuroforme.
- 2 DC de Z N O,
- 3 gouttes d'eugénol,
   et 0,05% d'oléorésine de tomate, colorant rouge contenant le Lycopène.
- Le PH du mélange A+B+C+D en solution P.P.I est voisin de 7.

La composition A,B,C,D se présente sous forme de base libre ou d'un sel pharmaceutiquement acceptable, avec oxyde de zinc comme catalyseur. La composition sous forme liquide, sous forme de pansement, de poudre, de pommade ou similaires ou sous forme de lotion, de spray. sera utilisée dans le domaine de la médecine, de la chirurgie, de la dentisterie, de la parodontologie et de l'implantologie, ainsi que dans le domaine vétérinaire et pour traiter des affections du type ulcérations et ses complications ou proliférations dysplasiques et des états tumoraux, bénins ou malins, chez l'homme ou chez l'animal.

**_Des adjonctions,** restant dans le cadre de l'invention,
sous forme de produits inertes peuvent être ajoutés à la préparation A+B+C+D, pour accentuer une propriété particulière : la dureté, la solubilité. Par exemple,
l'acide glycyrrhétique qui est un acide gras insaturé ; un agent viscosifiant ; un agent de texture qui est un oxyde de magnésium pour conférer l'élasticité ; la silice; les bioadhésifs tels que lesgommes ou celluloses ; un accélérateur de prise.

Les solutions ou suspensions peuvent contenir par exemple, des agents de suspension, tels que la méthylcellulose, la gomme adragante et l'alginate de sodium, des mouillants tels que la lécithine, le stéarate de polyxyéthylène et le mono-oléate de polyxyéthylène sorbitane.

La composition pharmaceutique peut être préparée par mélange de la substance active avec des diluants solides inertes, par exemple le lactose, l'amidon, la silice colloidale, la cellulose microcristalline ou des véhicules connus pharmaceutiquement acceptables.

Cette composition thérapeutique est utilisée en quantité et pour une période donnée thérapeutiquement efficace pour traiter de telles conditions.

### TROIS SOLUTIONS POSSIBLES DE REACTIONS ENTRE LE BUTOFORM, L'EUGENOL ET L'OXYDE DE ZINC :

L'oxygène de ZNO pourrait complexer les alcools, les phenols, en faisant des liaisons hydrogènes

Le zinc (ZN) est un anti oxydant qui complexe les amines, comme tout métal.

### Création de liaisons hydrogène :

### 1^{ère} hypothèse :

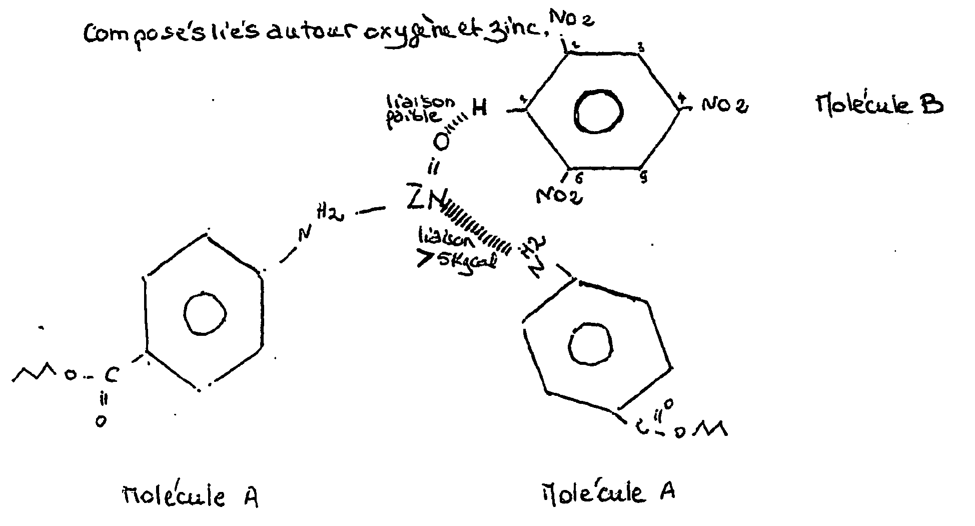

### 2^{ème} hypothèse

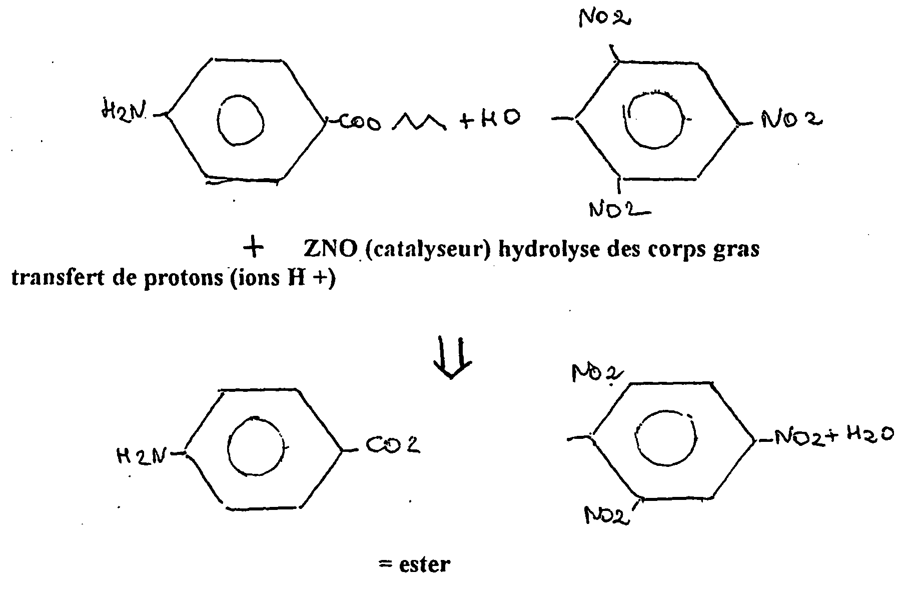

**Le ZN de la DNA polymérase, associé au magnésium du corps humain, (Mg) étant un cofacteur d'activités d'une multitude d'enzymes se placent au centre de la synthèse prothéique**

### 3eme HYPOTHESE :

### ZNO lèverait et diminuerait la barrière énergétique de l'ester.

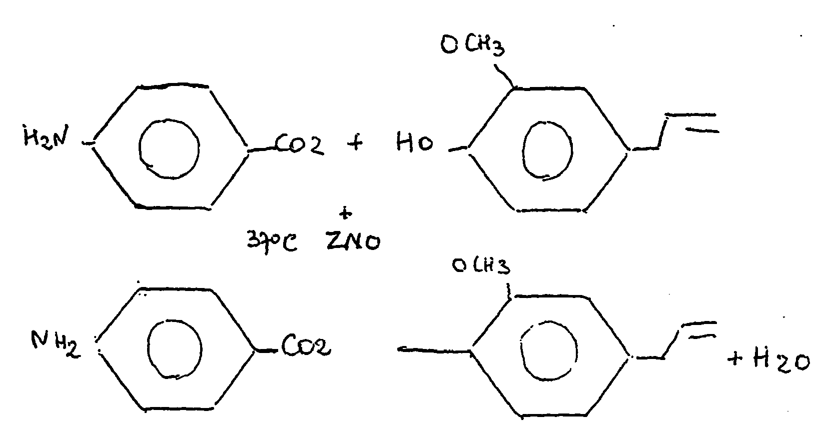

## Revendications

1. **COMPOSITION pharmaceutique,** comprenant l'association : **A+B+C+D, caractérisée en ce qu'**elle comprend :
Le **produit A** représente un anesthésique local appelé **Butoforme ou Scuroforme** sous forme de base libre, en mélange avec un **Eugénate** composé d'un **produit B : l'Eugénol** et un **produit C : l'Oxyde de zinc.**
A l'Eugenate, on ajoute un **produit D: le Lycopène,** contenu dans l'oléorésine de tomate. Cette association est additionnée d'excipients inertes, pharmaceutiquement acceptables, tels que le lactose, l'amidon, la silice colloïdale, la cellulose microcristalline.

2. **COMPOSITION de l'association A, B, C, D ,** selon la revendication n° 1 **caractérisée en ce qu'**elle contient une quantité de poids spécifique de 2 DC d'Oxyde de Zinc et 1 / 16 de CG de Butoforme ou de Scuroforme et une concentration de 3 gouttes d'Eugénol et 0,05% d'oléorésine de tomate contenant du lycopène. La composition a un PH 7.

3. **METHODE de préparation** de la composition selon la revendication n°1 et 2 du produit final A, B, C , D : des cristaux de Butoforme ou Scuroforme sont écrasés pour permettre leur dissolution dans l'Eugénol auxquels on ajoute l'Oxyde de Zinc, par malaxage jusqu'à obtenir une consistance mastic. On additionne 0,05% d'oléorésine de tomate contenant du lycopène pour son rôle antioxydant afin d'obtenir la coloration tissulaire souhaitée.

4. **UTILISATION de la composition** selon les revendications n°1 et 2 pour fabriquer un médicament destiné à obtenir une rémission de la douleur aigue ou chronique représentant le produit A+B+C+D contenant le **Butoforme** ou **le Scuroforme,** comme principe actif sous forme de base libre ou d'un sel pharmaceutiquement acceptable et **l'Oxyde de Zinc** en tant que catalyseur associé à **l'Eugénol,** additionné de colorant **Lycopène** pour son rôle d'antioxydant et pour améliorer l'esthétique tissulaire.

5. **UTILISATION de la composition** selon les revendications 1, 2 et 4, pour préparer un médicament destiné à améliorer la cicatrisation tissulaire et osseuse.

6. **UTILISATION de l'association A+B+C+D** selon les revendications 1 et 2 pour préparer un médicament présentant des propriétés préventives ou curatives à l'égard de certaines affections du type ulcérations et ses complications ou proliférations dysplasiques et des états tumoraux bénins ou malins, chez l'homme et chez l'animal.

## Claims

1. **Pharmaceutical COMPOSITION,** comprising of the association of **A+B+C+D,** wherein:
The product A is made up of a local anaesthetic called **Butoforme or Scuroforme** as a free base mixed with zinc oxide eugenol, the latter being made up of both **product B: Eugenol and product C: zinc oxide.**
**To the zinc oxide eugenol is added product D: Lycopene** which is contained in the oleoresin tomato. To this composition is added pharmaceutically acceptable inert excipients, such as lactose, starch, colloidale silicia, microcrystallin cellulose.

2. **COMPOSITION of the association A, B, C, D** as defined in claim n° 1 **characterized by** the fact it contains a quantity of specific weight of 2 DC of Zinc Oxide and 1/16 of CG of Butoforme or Scuroforme and a concentration of 3 drops of Eugenol and 0,05% of tomato oleoresin which contains lycopene. The composition has a pH level of 7.

3. **METHOD of preparation** of the composition as defined in claims n°1 and n°2 of the end product of A, B, C, D: Butoforme or Scuroforme crystals are crushed to enable their dissolution in Eugenol to which is added Zinc Oxide - this is mixed until a mastic consistence is obtained. 0,05% of tomato oleoresin which contains Lycopene is added for its antioxidant role in order to obtain the desired colouration of the tissue.

4. **USING the composition** as defined in claims n°1, n°2 and n°3 to make a medicine in order to remiss a sharp or chronical pain, represented by products A+B+C+D containing Butoforme or the **Scuroforme** as an active principle in the form of free base or a pharmaceutically acceptable salt and **Zinc Oxide** such as a catalyser associated with **Eugenol**, added to the colorant **Lycopene** for its antioxidant role and to improve aesthetic effect at the tissue level.

5. **USING the composition** as defined in claims 1, 2 and 4 to prepare a medicine which contributes to the improvement in tissue and bone scar tissue formation.

6. **USING the association** A+B+C+D as defined in claims 1 and 2 to prepare a medicine offering preventive or curative properties for certain affections such as ulcerations and its complications or dysplasic proliferations and its tumorous conditions benign or malignant in humans and in animals.

## Patentansprüche

1. **Pharmazeutische Zusammensetzung,** die die Verbindung A + B + C +D enthält, charakterisiert darin, dass sie Folgendes enthält:
**Das Produkt A** stellt ein örtliches Anästhetikum dar, **dasButoform** oder **Scuroform** heißt, in Form einer freien Base, vermischt mit einem **Eugenat,** welches sich zusammensetzt aus einem Produkt B: das **Eugenol** und einem Produkt C: **das Zinkoxid.** Dem Eugenat wird ein Produkt D, **dasLycopene,** welches in dem Harzöl von Tomaten oder einem **pharmazeutisch akzeptierten** Träger enthalten ist, beigefügt. Dieser pharmazeutischen Verbindung können träge ,pharmazeutisch akzeptierte, Substanzen wie zum Beispiel Laktose, Stärke, kolloidales Silizium und mikrokristalline Zellulose hinzugefügt werden.

2. **Die Zusammensetzung A, B, C, D,** ist laut Anspruch Nr. 1 **dadurch** charakterisiert, dass sie ein spezifisches Gewicht von 2 DC von Zinkoxid enthält sowie ein 1/16 von CG von Butoform oder Scuroform und ein Konzentrat von drei Tropfen Eugenol und 0,05% Harzöl von Tomaten, das Lycopene enthält. Die Zusammensetzung hat den pH-Wert 7.

3. **Vorbereitungsmethode der Zusammensetzung** gemäss Anspruch Nr. 1 und 2 des Endproduktes **A, B, C, D:** Kristalle von Butoform oder Scuroform werden zermahlt um ihre Auflösung im Eugenol zu ermöglichen. Diesen fügt man Zinkoxid durch Mischen hinzu, bis man eine Beschaffenheit von Spachtelmasse erhält. Man fügt 0,05 % Harzöl von Tomaten hinzu, das Lycopene als Anti-Oxydans enthält, um die gewünschte Gewerbefärbung zu erhalten.

4. **Verwendung der Zusammensetzung** laut Ansprüchen Nr. 1 und 2 um ein Medikament herzustellen, das dazu bestimmt ist, ein Nachlassen akuten oder chronischen Schmerzes zu erreichen, und das das Produkt A + B + C +D darstellt, welches **Butoform und Scuroform** enthält, als aktives Prinzip in Form von freier Base oder eines **pharmazeutisch akzeptierten** Salzes und von **Zinkoxid** als Katalysator, der verbunden ist mit Eugenol , dem der Farbstoff **Lycopene** als Anti-Oxydans hinzugefügt wird und um die Gewebeästhetik zu verbessern.

5. **Verwendung der Zusammensetzung** laut Ansprüchen Nr.1, 2 und 4 für die Herstellung eines Medikaments, das die Gewebe- und Knochenvernarbung verbessert.

6. **Verwendung der Verbindung A + B + C + D** laut Ansprüchen 1 und 2 um ein Medikament herzustellen, das vorbeugende und heilende Eigenschaften aufweist bei bestimmten Erkrankungen wie Geschwüren und damit verbundene Komplikationen wie dyplasische Verbreitungen und gutartige oder bösartige Tumore bei Mensch und Tier.
